Europäisches Patentamt

European Patent Office

Office européen des brevets

(19)

(11) **EP 0 493 274 B1**

## (12) FASCICULE DE BREVET EUROPEEN

(45) Date de publication et mention
de la délivrance du brevet:
**28.02.1996 Bulletin 1996/09**

(51) Int. Cl.⁶: **B01J 27/192**, C07C 47/22,
C07C 45/34

(21) Numéro de dépôt: **91420453.2**

(22) Date de dépôt: **16.12.1991**

(54) **Composition catalytique pour la préparation d'aldéhydes alpha,beta-insaturés par oxydation d'oléfines en phase gazeuse et procédé d'oxydation**

Katalysator zur Herstellung von Alpha, Beta-ungesättigten Aldehyden durch Oxydation von Olefinen in der Gasphase und Oxydationsverfahren

Catalyst for the preparation of alpha, beta unsaturated aldehydes by gas phase oxidation of olefins and oxidation process

(84) Etats contractants désignés:
**BE DE ES FR GB IT NL**

(30) Priorité: **20.12.1990 FR 9016388**

(43) Date de publication de la demande:
**01.07.1992 Bulletin 1992/27**

(73) Titulaire: **RHONE-POULENC CHIMIE**
**F-92408 Courbevoie Cédex (FR)**

(72) Inventeurs:
• **Legendre, Olivier**
**F-95220 Herblay (FR)**
• **Caillod, Jack**
**F-92100 Boulogne (FR)**

• **Jaeger, Philippe**
**F-95170 Deuil-la-Barre (FR)**

(74) Mandataire: **Le Pennec, Magali et al**
**F-92165 Antony Cédex (FR)**

(56) Documents cités:
EP-A- 0 078 150        FR-A- 2 202 729
FR-A- 2 364 061        US-A- 4 267 386
US-A- 4 298 763        US-A- 4 414 134

Remarques:
Le dossier contient des informations techniques présentées postérieurement au dépôt de la demande et ne figurant pas dans le présent fascicule.

## Description

La présente invention a pour objet une nouvelle composition catalytique pour la préparation d'aldéhydes $\alpha,\beta$-insaturés par oxydation d'oléfines en phase gazeuse ; elle concerne également le procédé de préparation d'aldéhydes $\alpha,\beta$-insaturés utilisant cette composition catalytique. L'invention concerne plus particulièrement une composition catalytique comprenant un support (ou noyau central) sous forme de billes pleines et solides, une phase catalytiquement active du type molybdate de bismuth et de fer, dopée par du potassium et du phosphore et, le cas échéant, par un ou plusieurs autres éléments métalliques ou non-métalliques, ledit support étant enrobé par une couche adhérente de ladite phase catalytiquement active.

Dans le brevet français FR-B-2 047 199 sont envisagés des catalyseurs d'oxydation répondant à la formule générale :

$$Ni_a \, Co_b \, Fe_c \, Bi_d \, L_e \, M_h \, Mo_f \, O_g$$

dans laquelle :

- L peut notamment représenter le phosphore
- M peut notamment représenter le potassium
- a et b sont compris entre 0 et 15 et la somme (a + b) entre 2 et 15
- c est compris entre 0,5 et 7
- d est compris entre 0,1 et 4
- e est compris entre 0 et 4
- f vaut 12
- g est compris entre 35 et 85
- h est compris entre 0,01 et 0,5

Ces catalyseurs sont préparés en formant une suspension en milieu aqueux à partir de divers précurseurs des constituants élémentaires du catalyseur, en ajoutant un support (tel un gel de silice) à ladite suspension semblable à une pâte, en chauffant à siccité pour obtenir un gâteau qui est alors traité à température élevée en présence d'air et d'oxygène. Les catalyseurs sont employés sous forme de grains ou de comprimés.

Ces catalyseurs, massiques et dilués sont productifs mais ils peuvent induire des difficultés lors de la conduite d'un procédé d'oxydation à l'échelle industrielle. En effet, en lit fixe, des températures ponctuelles élevées, qui peuvent être observées, sont à l'origine d'un emballement indésirable de la réaction.

Le brevet français FR-B-2 202 729 montre qu'il est avantageux de mettre en oeuvre des catalyseurs d'oxydation du propylène en acroléine préparés par enrobage, c'est-à-dire constitués d'une couche catalytiquement active de même composition mais déposée à la surface externe d'un support inerte d'au moins 20 micronmètres de diamètre au lieu de la diluer par un support introduit avec les sels métalliques. Il est alors possible de mieux contrôler le dégagement de chaleur de la réaction qui s'effectue dans des lits fixes.

Néanmoins, ce mode particulier de mise en forme du catalyseur nécessite qu'une part importante de celui-ci soit réservée au support inerte (66 % en masse du catalyseur fini, d'après le seul exemple du brevet). La part réservée à la phase active par rapport aux catalyseurs précédents simplement dilués est diminuée ce qui entraîne une baisse très pénalisante de l'activité des catalyseurs.

Ceci peut se traduire industriellement par l'obligation soit de recourir à des réacteurs plus gros pour conserver la capacité de production et des conditions opératoires identiques soit d'augmenter la température de réaction pour conserver la capacité de production et la taille du réacteur. Dans le premier cas l'inconvénient majeur est d'ordre économique. Dans le second cas, deux inconvénients se superposent : la sélectivité en acroléine sera plus faible et l'activité du catalyseur diminuera plus rapidement au cours du temps.

Le brevet américain US-A-4 298 763 préconise, pour l'oxydation du propylène en acroléine, une composition catalytique calcinée (phase active), répondant à la formule générale :

$$Mo_{12} \, Bi_{0,1-4} \, Fe_{0,5-6} M2_b \, M3_c \, M4_d \, M5_e \, O_x$$

dans laquelle :

- M2 est du nickel et/ou du cobalt
  b vaut de 2 à 12
- M3 peut être notamment K
  c vaut de 0,01 à 0,1 et de préférence, de 0,03 à 0,09
- M4 est P
  d vaut de 0 à 1 et de préférence, de 0,01 à 0,2

- M5 peut être In et/ou Na

    e vaut de 0 à 0,5 et de préférence, de 0,01 à 0,2
- x est le nombre d'atomes d'oxygène requis pour saturer

    les valences des autres constituants.

Cette phase active est déposée en couche d'une épaisseur de 150 à 1500 µm sur un support qui est aussi un noyau central d'un diamètre supérieur à 100 µm et d'une surface inférieure à 15 m²/g.

Le dépôt de la couche de matériau catalytique, calciné et pulvérulent, la dimension des particules étant comprise entre 0,1 et 300 µm, est réalisé en milieu humide, les particules de support étant vigoureusement agitées, des conditions opératoires contrôlées étant par ailleurs précisées.

La couche enrobant le noyau central de support représente au moins 50 % du poids du support soit au moins 33 % en poids du catalyseur fini et, au maximum 250 % du poids du support, soit au plus 71,4 % en poids du catalyseur fini.

Avant son utilisation dans l'oxydation des oléfines, le catalyseur enrobé est séché et, le cas échéant, calciné à une température de 400 à 700°C.

A supposer que ces catalyseurs présentent une activité catalytique satisfaisante, leur procédé de préparation de nature très complexe rend très difficile la tâche de l'homme de l'art qui vise un fonctionnement reproductible dudit procédé et l'assurance d'une qualité constante des catalyseurs ainsi préparés. En outre, il est bien connu de l'homme de l'art que, lorsque l'épaisseur de la couche enrobante croît pour dépasser de l'ordre de 20 % en poids par rapport au catalyseur, les propriétés mécaniques de ladite couche sont insuffisantes pour un usage des catalyseurs à l'échelle industrielle dans des réacteurs à lit fixe (cf.par exemple US-A-4 521 618).

Un besoin se fait donc sentir depuis de nombreuses années de pouvoir disposer de catalyseurs préparés par enrobage d'une phase catalytiquement active du type molybdate de bismuth et de fer, dopée par du potassium et du phosphore, offrant simultanément et durablement une activité catalytique et une sélectivité élevées, à l'échelle industrielle dans des réacteurs à lit fixe, lors de la préparation d'aldéhydes a,z-insaturés par oxydation d'oléfines en phase gazeuse et, en particulier lors de la préparation d'acroléïne par oxydation du propylène.

La présente invention a donc pour objet une composition catalytique comprenant :

- un support sous forme de billes pleines et solides de diamètre compris entre 0,5 et 6 min, et
- une phase catalytiquement active répondant à la formule générale ci-après :

$$A_a \, Mo_b \, W_c \, Bi_d \, Fe_e \, P_f \, K_g \, B_h \, C_i \, O_x$$

dans laquelle :
- A représente un atome de cobalt, nickel manganèse, magnésium et/ou plomb, et de préférence, de cobalt et/ou de nickel
- B représente un atome d'arsenic et/ou de bore,
- C représente un atome de métal alcalin différent du potassium et/ou un atome de métal alcalino-terreux différent du magnésium,
- a représente la somme des nombres d'atomes des éléments A et est compris entre 2 et 12 inclus ; lorsque A représente le cobalt seul, a est compris entre 8 et 10 inclus ;
- b est compris entre 10 et 12 inclus
- c est compris entre 0 et 2 inclus et le total (b + c) vaut 12
- d est compris entre 0,5 et 4 inclus
- e est compris entre 0,5 et 4 inclus
- f et g sont chacun compris entre 0,005 et 0,06 inclus et, de préférence, entre 0,01 et 0,03 inclus
- h représente la somme des nombres d'atomes des éléments B et est compris entre 0 et 4 inclus
- i représente la somme des nombres d'atomes des éléments C et est compris entre 0 et 0,5 inclus et
- x est le nombre d'atomes d'oxygène requis pour saturer les valences des autres constituants,

ledit support étant uniformément recouvert d'une couche adhérente de ladite phase catalytiquement active, et ladite phase catalytiquement active représente de 15 à 33 % en poids de la composition catalytique.

La présente invention a également pour objet un procédé d'oxydation d'oléfines en phase gazeuse mettant en oeuvre une telle composition catalytique et, plus particulièrement un procédé d'oxydation du propylène en acroléine.

La composition catalytique selon la présente invention comprend un support sous forme de billes pleines et solides de diamètre compris entre 0,5 et 6 mm. La valeur précise du diamètre des billes pourra être choisie par l'homme de l'art en fonction de la perte de charge admissible dans le réacteur. La nature du support n'est pas critique dès lors qu'il est chimiquement inerte vis-à-vis des réactifs dans les conditions réactionnelles choisies. A titre d'exemples de supports susceptibles de convenir à la préparation des compositions selon l'invention, on peut citer la silice, l'alumine, la silice-alumine, l'argile frittée, le carborandun, la magnésie et le silicate de magnésium. S'il est important que le support présente une rugosité de surface pour obtenir une résistance mécanique suffisante pour un usage en lit fixe sur une durée acceptable à l'échelle industrielle, la rugosité de surface, définie par la hauteur des aspérités rapportées au diamètre moyen des billes, peut varier dans de larges limites. De préférence, la rugosité ainsi définie est comprise entre 0,1 et 0,2.

La composition catalytique selon la présente invention comprend également une phase catalytiquement active à base de molybdate de bismuth et de fer, dopée par du potassium et du phosphore et, le cas échéant, par un ou plusieurs autres éléments métalliques ou non métalliques.

Ce type de phases catalytiquement actives est bien connu de l'homme de l'art et peut être préparé par tout moyen connu dans l'art antérieur tel le mélange de sels convenables des constituants élémentaires dans l'eau suivi de l'évaporation jusqu'à siccité, comme cela est décrit dans les brevets français FR-B-1 514 167 et FR-B-2 364 061 ; ou telle l'atomisation d'une suspension obtenue après mélange des sels convenables comme cela est proposé par le brevet américain US-A-4 298 763 et les demandes de brevet européen EP-A-25 715 et EP-A-77 675.

Selon une caractéristique essentielle de la composition selon l'invention le phosphore et le potassium sont présents chacun en quantité atomique comprise entre 0,005 et 0,06 pour 12 atomes de molybdène ou de molybdène et tungstène dans ladite phase.

La Demanderesse a en effet découvert de manière tout à fait inattendue que de meilleures activités catalytiques sont obtenues lorsque de faibles teneurs en dopants, phosphore et potassium, sont retenues. De préférence la teneur de chacun d'entre eux sera comprise entre 0,01 et 0,03. Pour obtenir une activité catalytique la plus élevée possible, il est avantageux de maintenir le rapport atomique P/K entre 0,3 et 3 inclus et, de préférence, entre 0,5 et 1,5 inclus.

Selon une autre caractéristique essentielle de la composition selon l'invention, ladite phase catalytiquement active représente de 15 à 33 % en poids de la composition totale et, de préférence de 20 à 30 % en poids. En dessous de 15 % en poids l'activité du catalyseur serait insuffisante à l'échelle industrielle et au dessus de 33 % en poids, la résistance mécanique au catalyseur serait trop aléatoire pour un usage industriel prolongé.

De préférence, A représente un atome de cobalt. Comme indiqué ci-avant, f/g est avantageusement compris entre 0,3 et 3 inclus et, de préférence entre 0,5 et 1,5 inclus.

La Demanderesse, suppose en outre qu'une partie de la bonne activité des catalyseurs selon l'invention peut être reliée à la présence dans la couche enrobante d'une phase cristallographique spécifique appelée "phase X", répondant à la stoechiométrie $Bi_3Fe_1Mo_2O_{12}$ et dont la détection peut être réalisée en soumettant une poudre, obtenue par attrition de la composition catalytique finie, à un examen par diffraction des rayons X.

Le spectre de diffraction X est le suivant :

| d(A) | Intensité visuelle relative |
|------|------------------------------|
| 3,17 | Très large |
| 3,14 | Très fort |
| 2,91 | Fort |
| 2,69 | Faible |
| 2,63 | Fort |
| 1,87 | Très très faible |

La préparation de la composition selon l'invention peut être réalisée de diverses manières et en particulier il est possible de préparer d'abord une phase catalytiquement active par des moyens connus dans l'art antérieur tel le mélange de sels convenables des constituants élémentaires dans l'eau suivi de l'évaporation jusqu'à siccité.

Les sels convenables les plus couramment employés dans l'art antérieur sont solubles dans l'eau et contiennent des anions et des cations qui peuvent être décomposés par la chaleur lors des étapes ultérieures. Ce sont par exemple l'heptamolybdate d'ammonium et le paratungstate d'ammonium pour le molybdène et le tungstène, les nitrates de cobalt, fer, nickel et bismuth pour les métaux. Les dopants sont aussi introduits sous forme de composés solubles et décomposables. Par exemple l'acide phosphorique à 85 %, l'arsine, la soude, la potasse, la magnésie ou les acétates, nitrates ou phosphates alcalins ou alcalino-terreux.

Une fois le mélange des sels réalisés, un précurseur peut être obtenu par la méthode dite d'évaporation. L'eau de la suspension obtenue est évaporée en chauffant entre 60 à 90°C sous agitation pendant le temps nécessaire à l'obtention d'une pâte non coulante. L'agitation et le chauffage sont alors arrêtés. La pâte ainsi obtenue, étalée sur une épaisseur de 2 cm environ est séchée sous air à 120°C pendant 16 heures. Le précurseur ainsi obtenu doit être calciné après avoir été coupé en morceaux de 1 à 2 cm. La calcination s'effectue en montant progressivement la température, 100 à 200°C par heure, en raison des risques liés à la décomposition exothermique du nitrate d'ammonium vers 230°C. La température est ensuite maintenue à une valeur stable entre 400 et 460°C pendant 6 heures puis le refroidissement est effectué en quelques heures. La phase active ainsi obtenue est alors broyée pour que sa granulométrie n'excède pas 400 micromètres.

Le précurseur peut aussi être obtenu selon une variante comprenant la précipitation avec addition d'ammoniaque en fin de mélange des sels pour faire remonter le pH vers environ 7, comme cela est proposé dans le brevet français FR-B-2 481 146. La solution d'ammoniaque contient entre 50 et environ 250 g d'ammoniac et elle ajoutée à une vitesse comprise entre environ 20 et environ 200 g d'ammoniac par heure et par litre de mélange. Il est préférable de chauffer ensuite la suspension entre 20 et 100°C pendant environ une heure pour parfaire la précipitation des espèces. Puis la suspension est filtrée. Le gateau de filtration est ensuite étalé sur une épaisseur inférieure à 2 cm et calciné selon les conditions décrites ci-avant dans le cadre de la méthode d'évaporation, pour donner la phase active. Dans cette variante, les dopants à base de sels solubles de phosphore et de potassium ne sont pas introduits dans la suspension de sels métalliques mais ultérieurement par imprégnation de la phase active "intermédiaire" ou du catalyseur "intermédiaire" ou, lors de l'enrobage ( Cf. EP-A-0 493 275).

Les catalyseurs sont obtenus par enrobage des phases actives, intermédiaires ou finies, broyées. Cette méthode classique consiste à déposer autour de billes inertes mais rugueuses une couche de phase active intermédiaire ou finie. Divers procédés d'enrobage sont décrits dans les brevets déjà cités. La variante préférée consiste à effectuer l'enrobage dans un drageoir cylindrique où l'on fait tourner 80 à 160 kg de billes inertes et rugueuses soigneusement dépoussiérées. Ces billes sont constituées avantageusement d'argile frittée à haute température mais il est possible d'utiliser tout autre support inerte et rugueux comme cela à déjà été indiqué. Dans le drageoir, on introduit ensuite 30 à 50 kg phase active et 8 à 15 litres de solution aqueuse d'un agent collant et éventuellement d'autres additifs. Dans une variante préférée, l'introduction de la phase active en poudre se fait simultanément à la pulvérisation de la solution aqueuse sur des billes préalablement humectées. Une fois les billes recouvertes de la totalité de la phase active, elles sont séchées par de l'air chaud, entre 80 et 150°C pendant 2 à 30 minutes, puis introduites dans des fours. La température de ces fours est montée linéairement en 3 à 15 heures à une valeur stable comprise entre 450 et 500°C. Puis le refroidissement s'effectue en 3 à 10 heures. Dans une variante préférée, une deuxième calcination est réalisée successivement et dans les mêmes conditions de variation de température que la première calcination.

La présente invention a également pour objet un procédé d'oxydation d'oléfines en phase gazeuse mettant en oeuvre une telle composition catalytique et, plus particulièrement un procédé d'oxydation du propylène en acroléine.

En général, le procédé d'oxydation est mis en oeuvre selon la technique du lit fixe dans un réacteur multitubulaire. Dans un mode de réalisation, chaque tube possède un diamètre intérieur de 15 à 30 mm et une longueur de 2 à 5 mètres, préférentiellement de 3 à 4 mètres et il est entouré d'un bain de fluide caloporteur permettant l'évacuation des calories dégagées par la réaction, par exemple un mélange eutectique de sels fondus. Les réactifs sont introduits gazeux dans le réacteur à une température comprise entre 100 et 250°C. Pour l'oxydation du propylène, ils sont constitués en général de 6 à 10 % molaire de propylène, de 50 à 65 % molaire d'air et de 25 à 40 % de vapeur d'eau. Il est possible de remplacer tout ou partie de la vapeur d'eau par des gaz inertes recyclés en fin d'opération. Ces gaz inertes contiennent de l'azote, de la vapeur d'eau et des oxydes de carbone. La pression des réactifs à l'entrée du réacteur est comprise entre 1 et 3 bars, de préférence légèrement supérieure à la pression atmosphérique pour tenir compte des pertes de charges dans le réacteur et dans le reste de l'installation, soit en pratique préférentiellement comprise entre 1,5 et 2,5 bars.

Pour l'oxydation du propylène, la température de bain de sels est comprise entre 280 et 360°C.

Les exemples ci-après illustrent la présente invention.

EXEMPLE 1 : Préparation d'une composition catalytique selon l'invention

a) Préparation du précurseur de la phase active.

Le précurseur est obtenu par réaction entre une solution (A) de nitrates de Co, Fe, Bi et K et une solution (B) d'heptamolybdate d'ammonium contenant de l'acide phosphorique.

La solution (A) de nitrates de Co, Fe, Bi et K dont le pH est inférieur à 1 est obtenue par mélange des 4 solutions préparées séparément comme suit :

. dans 50 ml d'eau désionisée, on dissous à température ambiante 116,4 g de nitrate de cobalt hydraté de formule $Co(NO_3)_2,6H_2O$ ; le pu de la solution ainsi obtenue est de 1,8.

. Dans 12,5 ml d'eau désionisée, on dissous à température ambiante 16,2 g de nitrate de fer hydraté de formule $Fe(NO_3)_2, 9H_2O$ ; le pH de la solution ainsi obtenue est de 0,7.

. Dans 14 ml d'eau désionisée, on ajoute 2 ml d'acide nitrique concentré de densité 1,4 g/l puis on ajoute à température ambiante 19,4 g de nitrate de bismuth hydraté de formule $Bi(NO_3)_3,5H_2O$ ; le pH de la solution ainsi obtenue est de 0.

. Dans 2,5 ml d'eau désionisé, on dissous à température ambiante 0,20 g de nitrate de potassium de formule $KNO_3$.

La solution d'heptamolybdate d'ammonium contenant l'acide phosphorique est obtenue en dissolvant à température ambiante 84,75 g d'heptamolybdate d'ammonium dans 380 ml d'eau désionisée à laquelle sont ajoutés 0,227 g d'acide phosphorique à 85 % dans 2 ml d'eau. Le pH de cette solution (B) est de 5,3.

Pour obtenir le précurseur de la phase active, on additionne, sous agitation, lentement (en 20 minutes) la solution (A) des nitrates dans la solution (B) d'hetamolybdate. Durant cette addition l'agitation est assurée par un agitateur à pales hélicoïdales tournant à 1100 tours par minute.

A la fin de l'addition, on obtient une suspension de couleur saumon qui est maintenue sous agitation pendant une demi-heure à température ambiante. Pendant cette agitation, le pH se stabilise vers 1,1.

On chauffe sous agitation vers 80°C pour évaporer l'eau ; au bout de deux heures, on obtient une pâte non coulante. L'agitation et le chauffage sont arrêtés. La pâte ainsi obtenue, étalée sur une épaisseur de 2 cm environ, est séchée sous air à 120°C pendant 48 heures. On obtient alors un solide qui est le précurseur de la phase active.

b) Préparation et conditionnement de la phase active.

Le solide ainsi obtenu est découpé en morceaux d'environ 1 cm et placé dans le four de calcination sur une épaisseur de 2 à 3 cm. La précalcination est effectuée à 400°C pendant 6 heures. La montée en température du four ne doit pas être trop rapide à cause de la réaction exothermique de décomposition du nitrate d'ammonium vers 230°C. La vitesse de montée en température est de l'ordre de 250°C par heure.

Le solide ainsi obtenu est la phase active, dont la composition est $Co_{10}Mo_{12}Fe_1Bi_1K_{0,05}P_{0,05}O_x$ ; il est broyé pour obtenir une granulométrie inférieure à 400 micromètres.

c) Enrobage du support par la phase active.

On ajoute 52 g de phase active broyée à une solution de 8 g de glucose dans 70 ml d'eau désionisée, à température ambiante. On chauffe l'ensemble à 70°C sous bonne agitation pendant 30 mn environ.

Dans un drageoir de 20 cm de diamètre contenant 210 g de billes d'argile frittée à 1100°C, et de rugosité de surface 0,3, de diamètre 4,8 mm et portées à 70°C, on verse la suspension précédente. On maintient la rotation du drageoir et la température vers 80°C jusqu'à évaporation complète de toute l'eau. Les billes ainsi obtenues sont ensuite séchées à 140°C pendant 2 heures. Elles sont ensuite placées dans un four de calcination sur une épaisseur de 2 à 3 cm puis calcinées à 480°C pendant 6 h sous atmosphère confinée. Cette première calcination entraîne l'élimination du glucose. Elle est suivie d'un refroidissement lent puis d'une seconde calcination à 480°C pendant également 6 heures. On obtient ainsi 259,5 g de catalyseur final sur lequel le taux massique de phase active est de 19,1 %.

EXEMPLE 2 : Préparation d'une première composition catalytique témoin

On opère comme décrit dans l'exemple 1, à ceci près que l'on introduit 0,285 g de pentoxyde de phosphore dans la solution contenant déjà l'heptamolybdate d'ammonium et 0,4 g de nitrate de potassium dans la solution contenant les nitrates métalliques.

La composition chimique de la phase active ainsi préparée est donc $Co_{10}Mo_{12}Fe_1Bi_1K_{0,1}P_{0,1}O_x$ et n'entre pas dans le cadre de la présente invention.

La suite des opérations est identique à celle de l'exemple 1. Le taux massique de phase active sur le catalyseur fini est de 19,1 %.

EXEMPLE 3 : Préparation d'une seconde composition catalytique témoin -

On opère comme décrit dans l'exemple 1, à ceci près que l'on introduit 1,4 g de pentoxyde de phosphore dans la solution contenant déjà l'heptamolybdate d'ammonium et 2,0 g de nitrate de potassium dans la solution contenant les nitrates métalliques.

La composition chimique de la phase active ainsi préparée est donc $Co_{10}Mo_{12}Fe_1Bi_1K_{0,5}P_{0,5}O_x$ et n'entre pas dans le cadre de la présente invention.

La suite des opérations est identique à celle de l'exemple 1. Le taux massique de phase active sur le catalyseur fini est de 18,7 %.

EXEMPLE 4 : Détermination de l'activité catalytique des diverses compositions

Les catalyseurs ainsi préparés sont testés dans l'oxydation ménagée du propylène en acroléine dans un réacteur de diamètre interne 21 mm et de 50 cm de haut contenant 100 ml de catalyseur.

Le mélange réactionnel introduit dans le réacteur chauffé par un bain de sable contient en pourcentage volumique : 7 % de propylène, 57 % d'air et 36 % de vapeur d'eau. Le débit de propylène est ajusté pour obtenir une charge d'environ 166 g de propylène par heure et par litre de catalyseur. La pression de sortie du réacteur est régulée à 1,8 bar absolu.

Les effluents du réacteur contiennent un mélange gazeux d'azote, d'oxygène, de vapeur d'eau, de propylène, d'acroléïne, d'acide acrylique, d'acide acétique, d'acétaldéhyde, de monoxyde et de dioxyde de carbone et d'autres impuretés en faible quantité. Des chromatographes en phase gaz permettent de déterminer les proportions en chacun de ces produits et donc de calculer les performances catalytiques, c'est-à-dire :

Le taux de transformation, noté $X_g$

$$X_g = \frac{\text{Nombre de moles de propylène disparu}}{\text{Nombre de moles de propylène á l'entrée}} \times 100$$

La sélectivité en produit i, notée $S_i$

$$S_i = \frac{\text{Nombre de moles de produit i formé}}{\text{Nombre de moles de propylène transformé}} \times 100$$

et $R_i$ qui est le rendement en produit i. Le rendement est le produit de la conversion $X_g$ par la sélectivité en produit i : $R_i = X_g \times S_i$

Les résultats obtenus sur les catalyseurs des exemples 1 à 3 sont reportés dans le tableau I ci-après :

TABLEAU I

| Ex | Dopants | Taux Massique (%) | Température de bain °C | Xg (%) | Sacroléine (%) | Sacrylique (%) | Sco-co2 (%) | Racroléine (%) |
|----|---------|---------|---------|--------|-----------|-----------|-------------|-----------|
| 1 | K0,05P0,05 | 19,1 | 351 | 92,2 | 82,5 | 7,6 | 3,7 | 76,0 |
| 2 | K0,1P0,1 | 19,1 | 360 | 87,3 | 85,5 | 6.5 | 2,0 | 74,6 |
| 3 | K0,5P0,5 | 18,7 | 349 | 43,8 | 89,9 | 2,1 | 0,9 | 39.4 |

Il apparaît clairement que l'augmentation des teneurs en dopants phosphore et potassium ajoutés en même quantité atomique diminue l'activité de ces catalyseurs, toutes choses égales par ailleurs.

Il faut donc soit augmenter la température de bain dans lequel est plongé le réacteur contenant le catalyseur pour tenter de maintenir la valeur de la conversion du propylène, comme cela est le cas lorsque la teneur en dopants double de $K_{0,05}P_{0,05}$ à $K_{0,1}P_{0,1}$, soit tolérer une conversion bien plus faible du propylène pour conserver constante la température du bain, ce qui n'est pas acceptable à l'échelle industrielle.

Il est à noter que la valeur du rendement en acroléine obtenu sur la composition catalytique de l'exemple 1, selon l'invention, en une seule passe est tout à fait remarquable.

EXEMPLE 5 : Préparation d'une composition catalytique témoin

On opère comme dans l'exemple 1, sauf que l'on introduit 0,285 g de pentoxyde de phosphore dans la solution contenant déjà l'heptamolybdate d'ammonium et 0,20 g de nitrate de potassium dans la solution contenant les nitrates métalliques.

La composition chimique de la phase active ainsi préparée est donc $Co_{10}Mo_{12}Fe_1Bi_1K_{0,05}P_{0,1}O_x$ et n'entre pas dans la cadre de la présente invention.

La suite des opérations est identique à celle de l'exemple 1. Le taux massique de phase active sur le catalyseur fini est de 18,9 %.

EXEMPLE 6 : Détermination de l'activité catalytique de la composition préparée à l'exemple 5

Le catalyseur ainsi préparé est testé dans l'oxydation ménagée du propylène en acroléine dans les mêmes conditions que celles décrites précédemment dans l'exemple 4. Les résultats des mesures effectuées sur le catalyseur de l'exemple 5 comparativement à celles effectuées sur le catalyseur 1 sont reportés dans le tableau II ci-dessous :

TABLEAU II

| Ex | Dopants | Taux Massique (%) | Température de bain °C | $X_g$ (%) | Sacroléine (%) | Sacrylique (%) | Sco-co2 (%) | Racroléine (%) |
|---|---|---|---|---|---|---|---|---|
| 1 | K0,05P0,05 | 19,1 | 351 | 92,2 | 82,5 | 7,6 | 3.7 | 76,0 |
| 1 | K0,05P0,05 | 19,1 | 340 | 87.2 | 85,7 | 5,6 | 2,5 | 74.7 |
| 5 | K0,05P0,1 | 18,9 | 361 | 85,7 | 84,8 | 6,9 | 2.2 | 72,7 |

Il apparaît donc que l'augmentation du rapport atomique Phosphore sur Potassium diminue considérablement l'activité catalytique. Le catalyseur de l'exemple 5, même porté à une température de bain de 361°C, converti encore moins le propylène que le catalyseur dopé au rapport atomique 1 à la température de 340°C.

EXEMPLE 7 : Préparation d'une composition catalytique entrant dans le cadre de la présente invention -

On opère comme dans l'exemple 1, sauf que l'on introduit 0,114 g d'acide phosphorique à 85 % dans la solution contenant déjà l'heptamolybdate d'ammonium et 0,20 g de nitrate de potassium dans la solution contenant les nitrates métalliques.

La composition chimique de la phase active ainsi préparée est donc $Co_{10}Mo_{12}Fe_1Bi_1K_{0,05}P_{0,025}O_x$.

La suite des opérations est identique à celle de l'exemple 1. Le taux massique de phase active sur le catalyseur fini est de 19,2 %.

EXEMPLE 8 : Préparation d'une composition catalytique entrant dans le cadre de la présente invention -

Cette composition diffère de celle décrite à l'exemple 7 ci-avant par le fait qu'on n'utilise que 140 g de support au lieu de 210 g.

La suite des opérations est identique à celle de l'exemple 7. Le taux massique de phase active sur le catalyseur fini est de 23,9 %.

EXEMPLE 9 :

On opère exactement comme dans l'exemple 7, sauf que l'on n'utilise que 105 g de support au lieu de 210 g et 59 g de phase active au lieu de 50 g.

La suite des opérations est identique à celle de l'exemple 7. Le taux massique de phase active sur le catalyseur fini est de 33,0 %.

EXEMPLE 10 : Influence du taux de phase active sur l'activité catalytique

Les catalyseurs ainsi préparés sont testés dans l'oxydation ménagée du propylène en acroléine dans les mêmes conditions que celles décrites précédemment dans l'exemple 4, sauf en ce qui concerne le débit des réactifs qui est augmenté. La charge en propylène est portée de 166 à 250 g/h/litre. Les résultats des mesures effectuées sur les catalyseurs des exemples 8 et 9 comparativement à celles effectuées sur le catalyseur de l'exemple 7 sont reportés dans le tableau III ci-dessous :

TABLEAU III

| Ex | Dopants | Taux Massique (%) | Température de bain °C | Xg (%) | Sacroléine (%) | Sacrylique (%) | Sco-co2 (%) | Racroléine (%) |
|---|---|---|---|---|---|---|---|---|
| 7 | K0,05P0,025 | 19,2 | 400 | 95,8 | 64,7 | 25,9 | 4,6 | 62,0 |
| 8 | K0,05P0,025 | 23,9 | 370 | 90,9 | 82,0 | 10,8 | 2,1 | 74,5 |
| 9 | K0,05P0,025 | 33,0 | 346 | 95,0 | 79,7 | 11,5 | 2,9 | 76,1 |

Il apparaît donc que l'augmentation de la teneur massique en phase active sur le catalyseur fini, toutes choses égales par ailleurs, se traduit par la possibilité de travailler à une température de bain plus faible. Cela se traduit aussi par des rendements en acroléine en augmentation sensible.

EXEMPLE 11 : Influence du taux de phase active sur la résistance mécanique des catalyseurs -

La résistance mécanique des catalyseurs finis est déterminée par un test d'attrition comme suit :

100 g de catalyseur fini sont introduits dans un tambour en plexiglass® de diamètre extérieur 200 mm et de largeur 40 mm fixé sur l'axe horizontal d'un moteur tournant à 10 tours par minute. A l'intérieur du tambour sont fixés, à intervalle régulier, 6 aubes planes en plexiglass® de 45 mm de long et de 40 mm de large, inclinées à 40 degrés par rapport au diamètre passant par leur base de fixation.

Le sens de rotation du tambour est tel que, si l'on représente le vecteur de la vitesse tangentielle du tambour en un point de fixation de l'une quelconque des aubes, celui-ci fasse un angle de 50 degrés avec l'aube.

Le tambour est mis en rotation pendant 5 minutes, puis les billes sont retirées du tambour et pesées après tamisage pour séparer les fines. La masse ainsi déterminée est $m_5$. L'appareil est dépoussiéré, puis les billes sont réintroduites. Une nouvelle pesée est effectuée au bout de 10 minutes, soit une masse $m_{15}$.

Le taux d'attrition est défini comme la proportion de phase active enlevée par le dispositif d'attrition. Il est calculé par rapport au taux T de phase active de la façon suivante :

Après 5 minutes, le taux d'attrition, en %, vaut $(100-m_5) \times 100/T$

Après 15 minutes cumulée d'attrition, le taux vaut $(100-m_{15}) \times 100/T$ .

Les mesures d'attrition effectuées sur les catalyseurs des exemples 7, 8 et 9 à teneur en phase active croissante, toutes choses égales par ailleurs, sont reportées dans le tableau IV ci-après :

TABLEAU IV

| Exemple | Dopants | Teneur T (%) | Taux d'attrition à 5 mn (%) | Taux d'attrition à 15 mn (%) |
|---------|---------|--------------|------------------------------|-------------------------------|
| 7 | K0,05P0,025 | 19,2 | 0,62 | 1,82 |
| 8 | K0,05P0,025 | 23,9 | 1,52 | 5,32 |
| 9 | K0,05P0,025 | 33,0 | 3,64 | 12,6 |

Il apparaît qu'effectivement, l'accroissement de la teneur en phase active se traduit bien par une diminution de la résistance mécanique.

**Revendications**

1. Composition catalytique comprenant :

- un support sous forme de billes pleines et solides de diamètre compris entre 0,5 et 6 mm, et
- une phase catalytiquement active de formule générale suivante :

$$A_a \, Mo_b \, W_c \, Bi_d \, Fe_e \, P_f \, K_g \, B_h \, C_i \, O_x$$

dans laquelle :

- A représente un atome de cobalt, nickel, manganèse, magnésium et/ou plomb,
- B représente un atome d'arsenic et/ou de bore,
- C représente un atome de métal alcalin différent du potassium et/ou un atome de métal alcalino-terreux différent du magnésium,
- a représente la somme des nombres d'atomes des éléments A et est compris entre 2 et 12 inclus ; lorsque A représente le cobalt seul, a est compris entre 8 et 10 inclus;
- b est compris entre 10 et 12 inclus
- c est compris entre 0 et 2 inclus et le total (b+c) vaut 12
- d est compris entre 0,5 et 4 inclus
- e est compris entre 0,5 et 4 inclus
- f et g sont chacun compris entre 0,005 et 0,06 inclus
- h représente la somme des nombres d'atomes des éléments B et est compris entre 0 et 4 inclus
- i représente la somme des nombres d'atomes des éléments C et est compris entre 0 et 0,5 inclus et
- x est le nombre d'atomes d'oxygène requis pour saturer les valences des autres constituants.

ledit support étant uniformément recouvert d'une couche adhérente de ladite phase catalytiquement active, et ladite phase catalytiquement active représente de 15 à 33 % en poids de la composition catalytique.

2. Composition selon la revendication 1, caractérisée en ce que le phosphore et le potassium sont présents chacun en quantité atomique comprise entre 0,01 et 0,03 pour 12 atomes de molybdène ou de molybdène et de tungstène.

3. Composition selon l'une des revendications 1 ou 2, caractérisée en ce que la phase catalytiquement active représente de 20 à 30 % en poids de la composition catalytique.

4. Composition selon l'une quelconque des revendications précédentes, caractérisée en ce que le rapport atomique P/K est compris entre 0,3 et 3 inclus.

5. Composition selon l'une quelconque des revendications précédentes, caractérisée en ce que le rapport atomique P/K est compris entre 0,5 et 1,5 inclus.

6. Composition selon l'une quelconque des revendications précédentes, caractérisée en ce que le support présente un rugosité de surface (hauteur des aspérités rapportée au diamètre moyen des billes) compris entre 0,1 et 0,2 inclus.

7. Composition selon l'une quelconque des revendications précédentes, caractérisée en ce que A représente un atome de cobalt.

8. Composition selon l'une quelconque des revendications précédentes, caractérisée en ce que la phase catalytiquement active renferme la phase cristallographique "phase X", répondant à la stoechiométrie $Bi_3Fe_1MO_2O_{12}$ et dont le spectre de diffraction X est le suivant :

| d(A) | Intensité visuelle relative |
|---|---|
| 3,17 | Très large |
| 3,14 | Très fort |
| 2,91 | Fort |
| 2,69 | Faible |
| 2,63 | Fort |
| 1,87 | Très très faible |

9. Procédé d'oxydation d'oléfines en aldéhydes $\alpha$, $\beta$- insaturés, en phase gazeuse caractérisé en ce que la réaction d'oxydation est conduite en présence d'une composition catalytique selon l'une quelconque des revendications précédentes.

10. Procédé d'oxydation du propylène en acroléine, en phase gazeuse caractérisé en ce que la réaction d'oxydation est conduite en présence d'une composition catalytique selon l'une quelconque des revendications 1 à 8.

**Claims**

1. Catalytic composition comprising:

- a support in the form of entire and solid spheres of diameter between 0.5 and 6 mm, and
- a catalytically active phase of the following general formula

$$A_a\ Mo_b\ W_c\ Bi_d\ Fe_e\ P_f\ K_g\ B_h\ C_i\ O_x$$

in which:

- A represents an atom of cobalt, nickel, manganese, magnesium and/or lead,
- B represents an atom of arsenic and/or of boron.
- C represents an atom of alkali metal other than potassium and/or an atom of alkaline earth metal other than magnesium,

- a represents the sum of the numbers of atoms of the elements A and is between 2 and 12 inclusive; when A represents cobalt alone, a is between 8 and 10 inclusive,
- b is between 10 and 12 inclusive,
- c is between 0 and 2 inclusive and the total (b + c) has a value of 12,
- d is between 0.5 and 4 inclusive,
- e is between 0.5 and 4 inclusive,
- f and g are each between 0.005 and 0.06 inclusive,
- h represents the sum of the numbers of atoms of the elements B and is between 0 and 4 inclusive,
- i represents the sum of the numbers of atoms of the elements C and is between 0 and 0.5 inclusive and
- x is the number of atoms of oxygen required to saturate the valencies of the other constituents,

the said support being uniformly covered with an adherent layer of the said catalytically active phase, and the said catalytically active phase representing between 15 and 33% by weight of the catalytic composition.

2. Composition according to claim 1, characterized in that the phosphorus and the potassium are each present in an atomic quantity between 0.01 and 0.03 per 12 atoms of molybdenum or of molybdenum and of tungsten.

3. Composition according to claim 1 or 2, characterized in that the catalytically active phase represents between 20 and 30% by weight of the catalytic composition.

4. Composition according to any one of the preceding claims, characterized in that the atomic ratio P/K is between 0.3 and 3 inclusive.

5. Composition according to any one of the preceding claims, characterized in that the atomic ratio P/K is between 0.5 and 1.5 inclusive.

6. Composition according to any one of the preceding claims, characterized in that the support has a surface roughness (height of the unevennesses relative to the mean diameter of the spheres) between 0.1 and 0.2 inclusive.

7. Composition according to any one of the preceding claims, characterized in that A represents an atom of cobalt.

8. Composition according to any one of the preceding claims, characterized in that the catalytically active phase contains the crystallographic phase, "phase X", corresponding to the stoichiometry $Bi_3Fe_1Mo_2O_{12}$ and whose X-ray diffraction spectrum is the following:

| d(A) | Relative visual intensity |
|---|---|
| 3.17 | very large |
| 3.14 | very strong |
| 2.91 | strong |
| 2.69 | weak |
| 2.63 | strong |
| 1.87 | very very weak |

9. Process for the oxidation of olefins to $\alpha,\beta$-unsaturated aldehydes in the gaseous phase, characterized in that the oxidation reaction is conducted in the presence of a catalytic composition according to any one the preceding claims.

10. Process for the oxidation of propylene to acrolein in the gaseous phase, characterized in that the oxidation reaction is conducted in the presence of a catalytic composition according to any one of claims 1 to 8.

**Patentansprüche**

1.  Katalytische Zusammensetzung, umfassend:

    -   einen Träger in Form von vollen und festen Kugeln mit einem zwischen 0,5 und 6 mm eingeschlossenen Durchmesser und
    -   eine katalytisch aktive Phase der folgenden allgemeinen Formel:

    $$A_a Mo_b W_c Bi_d Fe_e P_f K_g B_h C_i O_x$$

    in der

    -   A ein Kobalt-, Nickel-, Mangan-, Magnesium- und/oder Bleiatom darstellt,
    -   B ein Arsen- und/oder Boratom darstellt,
    -   C ein von Kalium verschiedenes Alkalimetallatom und/oder ein von Magnesium verschiedenes Erdalkalimetallatom darstellt,
    -   a die Summe der Zahl der Atome der Elemente A darstellt und zwischen 2 und 12 einschließlich liegt; wenn A nur Kobalt darstellt, liegt a zwischen 8 und 10 einschließlich;
    -   b zwischen 10 und 12 einschließlich liegt,
    -   c zwischen 0 und 2 einschließlich liegt und die Summe (b+c) 12 ist,
    -   d zwischen 0,5 und 4 einschließlich liegt,
    -   e zwischen 0,5 und 4 einschließlich liegt,
    -   f und g jeweils zwischen 0,005 und 0,06 einschließlich liegen,
    -   h die Summe der Anzahl der Atome der Elemente B darstellt und zwischen 0 und 4 einschließlich liegt,
    -   i die Summe der Atome der Elemente C darstellt und zwischen 0 und 0,5 einschließlich liegt und
    -   x die Zahl der Sauerstoffatome ist, die erforderlich ist, um die Valenzen der anderen Bestandteile abzusättigen,

    wobei der Träger gleichförmig mit einer anhaftenden Schicht der katalytisch aktiven Phase bedeckt ist und die katalytisch aktive Phase 15 bis 33 Gew.-% der katalytischen Zusammensetzung darstellt.

2.  Zusammensetzung nach Anspruch 1, dadurch gekennzeichnet, daß der Phosphor und das Kalium jeweils in einer Atommenge vorhanden sind, die zwischen 0,01 und 0,03 pro 12 Atome Molybdän oder Molybdän und Wolfram eingeschlossen liegt.

3.  Zusammensetzung nach einem der Ansprüche 1 oder 2, dadurch gekennzeichnet, daß die katalytisch aktive Phase 20 bis 30 Gew.-% der katalytischen Zusammensetzung darstellt.

4.  Zusammensetzung nach irgendeinem der vorangehenden Ansprüche, dadurch gekennzeichnet, daß das Atomverhältnis P/K zwischen 0,3 und 3 einschließlich liegt.

5.  Zusammensetzung nach irgendeinem der vorangehenden Ansprüche, dadurch gekennzeichnet, daß das Atomverhältnis P/K zwischen 0,5 und 1,5 einschließlich liegt.

6.  Zusammensetzung nach irgendeinem der vorangehenden Ansprüche, dadurch gekennzeichnet, daß der Träger eine Oberflächenrauheit (Höhe der Unebenheiten bezogen auf den mittleren Durchmesser der Kugeln) zwischen 0,1 und 0,2 einschließlich aufweist.

7.  Zusammensetzung nach irgendeinem der vorangehenden Ansprüche, dadurch gekennzeichnet, daß A ein Kobaltatom darstellt.

8.  Zusammensetzung nach irgendeinem der vorangehenden Ansprüche, dadurch gekennzeichnet, daß die katalytisch aktive Phase die kristallographische Phase "Phase X" einschließt, die der Stöchiometrie $Bi_3 Fe_1 Mo_2 O_{12}$ entspricht und deren Röntgen-Beugungsspektrum das folgende ist:

| d(A) | Visuelle relative Intensität |
|------|------------------------------|
| 3,17 | sehr breit |
| 3,14 | sehr stark |
| 2,91 | stark |
| 2,69 | schwach |
| 2,63 | stark |
| 1,87 | sehr sehr schwach |

9. Verfahren zur Oxidation von Olefinen zu $\alpha,\beta$-ungesättigten Aldehyden in der Gasphase, dadurch gekennzeichnet, daß die Oxidationsreaktion in Gegenwart einer katalytischen Zusammensetzung nach irgendeinem der vorangehenden Ansprüche durchgeführt wird.

10. Verfahren zur Oxidation von Propylen zu Acrolein in der Gasphase, dadurch gekennzeichnet, daß die Oxidationsreaktion in Gegenwart einer katalytischen Zusammensetzung nach irgendeinem der Ansprüche 1 bis 8 durchgeführt wird.